# FASCICULE DE BREVET EUROPEEN

(11) **EP 2 826 461 B1**
(45) Date de publication et mention de la délivrance du brevet: **01.06.2016**
(21) Numéro de dépôt: 14180383.3
(22) Date de dépôt: 14.11.2005
(51) Int. Cl.: A61K 8/42, A61Q 5/04, A61K 8/43

(54) **Procédé de défrisage des fibres kératiniques avec un moyen de chauffage et un agent dénaturant**
Verfahren zum Glätten von Keratinfasern mit einem Heizmittel und einem Denaturierungsmittel
Method for straightening keratin fibres using a heating means and a denaturing agent

(30) Priorité: 26.11.2004 FR 0452775
(43) Date de publication de la demande: 21.01.2015
(62) Demande divisionnaire de: 05292406.5
(73) Titulaire: L'Oréal, 75008 Paris (FR)
(72) Inventeur: Radisson, Xavier, 92600 Asnières sur Seine (FR); Barbarat, Philippe, 92270 Bois Colombes (FR); Malle, Gérard, 77580 Villiers s/Morin (FR); Diridollou, Stéphane, Chicago, IL 60657 (US)
(74) Mandataire: Kromer, Christophe

(56) Documents cités:
- EP-A1- 1 468 667
- EP-A1- 1 532 963
- EP-A1- 1 535 598
- EP-A2- 0 100 901
- GB-A- 1 466 622
- US-A- 1 861 040
- DATABASE CAPLUS [Online] CHEMICAL ABSTRACTS SERVICE, COLUMBUS, OHIO, US; KOBAYASHI, MIKIO ET AL.: "hair straightening or wave-setting compositions containing protein denaturants", XP002338918, extrait de CAPLUS Database accession no. 2003:585180 & JP 2003 212737 A (HOYU CO LTD) 30 juillet 2003 (2003-07-30)
- DATABASE EPODOC [Online] EUROPEAN PATENT OFFICE, THE HAGUE, NL; 20 janvier 1998 (1998-01-20), Kajino Takayoshi et al.: "Hair treatment agent composition", XP002732716, Database accession no. JP-17653196-A & JP H10 17441 A (KAO CORP) 20 janvier 1998 (1998-01-20)
- R Mcmullen ET AL: "Thermal degradation of hair. I. Effect of curling irons", J.Cosmet.Sci., vol. 49 1 juillet 1998 (1998-07-01), pages 223-244, XP055154260, Extrait de l'Internet: URL:http://journal.scconline.org/pdf/cc199 8/cc049n04/p00223-p00244.pdf [extrait le 2014-11-21]

## Description

L'invention a pour objet un procédé de défrisage des fibres kératiniques avec un moyen de chauffage et au moins un agent dénaturant.

Par *"fibres kératiniques",* on entend, des fibres d'origine humaine ou animale telles que les cheveux, les poils, les cils, la laine, l'angora, le cachemire ou la fourrure. L'invention référence aux cheveux.

Le terme *"défrisage"* englobe, selon l'invention, le défrisage, de cheveux caucasiens ou africains. Le terme «*défriser»* englobe, selon l'invention, défriser, des cheveux caucasiens ou africains.

On entend par *«agent dénaturant»,* un composé organique ou minéral possédant à la fois au moins un site donneur d'électrons à caractère basique ou nucléophile et au moins un site accepteur d'électrons à caractère acide ou électrophile, interagissant avec les liaisons faibles de la kératine.

Selon l'invention, un agent dénaturant est un composé capable de diminuer le pouvoir rotatoire d'une protéine modèle telle que par exemple la bovine sérum albumine d'au moins 7° et/ou 5° à 579nm, les mesures étant effectuées après 3h d'incubation à 37°C, à l'aide d'un polarimètre, tel que décrit dans Biochemistry 2(1), 47-57, 1963 :
- soit dans un tampon TRIS 0.05M pH 7,6
- soit dans une solution d'urée 5,45M lorsque la solubilité du composé est insuffisante dans le tampon TRIS 0.05M pH 7,6.

Le composé est considéré comme agent dénaturant selon l'invention si la diminution du pouvoir rotatoire est d'au moins 7° dans le tampon TRIS 0.05M pH 7,6 et/ou d'au moins 5° dans la solution d'urée 5,45M.

Par *«liaisons faibles de la kératine»,* on entend l'ensemble des liaisons non covalentes telles que :
- les liaisons salines résultant d'interactions coulombiennes entre les groupes fonctionnels présents au niveau des chaînes latérales des acides aminés
- les liaisons hydrogènes qui s'établissent entre les acides aminés par l'intermédiaire notamment des atomes d'oxygène et d'hydrogène
- les liaisons hydrophobes qui résultent de la tendance des chaînes non polaires des acides aminés à s'associer pour minimiser les contacts avec l'eau

Par «*moyen de chauffage»,* on entend tout moyen permettant de chauffer les fibres kératiniques à une température d'au moins 110°C tels que les fers chauffants, par exemple les fers plats ou ronds, les générateurs de micro-ondes, les sources de rayonnement infra-rouge.

Deux techniques sont utilisées pour obtenir une déformation permanente des cheveux. Elles sont basées sur une rupture des liaisons covalentes disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée. Cette technique permet indifféremment de réaliser, soit l'ondulation des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ▪ La première réaction consiste en une béta-élimination sur la cystine provoquée par un ion hydroxyde, conduisant à la rupture de cette liaison et à la formation de déhydro-alanine. 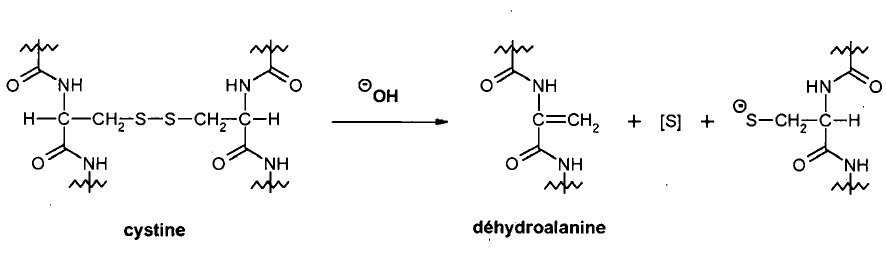
   ▪ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

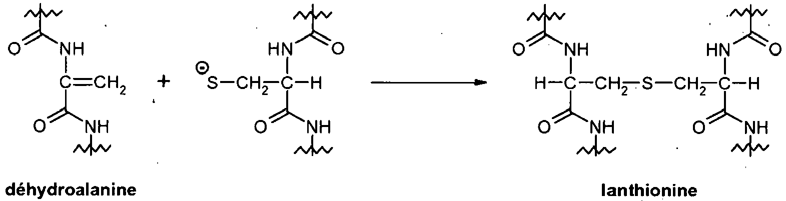

Par rapport à la première technique mettant en oeuvre un agent réducteur, cette technique de lanthionisation, ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente ("hair waving"). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.

Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot" base " ici utilisé n'ayant pas la signification d'agent basique au sens chimique. Lorsque la crème protectrice est associée à l'hydroxyde en une seule composition, celle-ci est généralement appelée " no-base ", par opposition à l'appellation ci-dessus. Cette technologie " no-base" est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais "lye relaxers" et celles n'en contenant pas sont dénommées " no-lye relaxers ".

Les principales formules défrisantes dites " no-lye " mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium ("lye") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas utilisées pour la permanente ("hair waving") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour le défrisage de compositions nettement moins agressives pour le cheveu.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, aussi bien pour la mise en forme que pour le lissage.

Il a également été proposé dans de très nombreux brevets d'associer des agents réducteurs habituels (thiols ou sulfites ou bisulfites) avec de l'urée ou des alkylurées pour diminuer l'irritation et les dommages causés aux cheveux aussi bien pour la mise en forme que pour le défrisage. On citera par exemple :
- la demande CA 1315204 qui décrit une composition contenant du thioglycolate d'ammonium (5,5-11,5%) et de l'urée ou une monoalkylurée (1-3%) pour la mise en forme des cheveux,
- la demande US 3847165 qui décrit une composition contenant du thioglycolate d'ammonium (1,2-1,4M) et de l'urée (2,0-2,7M) pour la mise en forme des cheveux à un pH acide,
- la demande NL 6410355 qui décrit une composition contenant un sulfite (0,8-1,5M) et de l'urée (0,6-3,0M) pour la mise en forme et le défrisage des cheveux,
- la demande JP 2000/229819 qui décrit une composition contenant un sulfite ou bisulfite (0,5-15%), de l'urée (0,5-15%) et un alcool (éthanol et/ou isopropanol, 1-30%) pour la mise en forme et le défrisage des cheveux,

Il a également été proposé dans de très nombreux brevets d'associer des hydroxydes, servant d'actif de lanthionisation, avec certains additifs servant généralement à protéger les cheveux. On citera, à titre d'exemple:
- la demande WO2002/003937, qui décrit une composition contenant des monosaccharides en C3-C5,
- la demande WO2001/064171, qui décrit une composition contenant des agents complexants,
- le brevet US5641477, qui décrit une composition contenant un hydrolysat d'amidon hydrogéné,
- la demande WO02085317, qui décrit une composition contenant des nucléophiles organiques qui réagissent lors de la deuxième étape avec la déhydro-alanine formée avec des hydroxydes, pour conduire à de nouveaux pontages.

Si toutes ces propositions conduisent à des améliorations plus ou moins marquées, elles ne permettent pas de diminuer de façon suffisante les dégâts liés à la causticité même des hydroxydes.

Comme indiqué précédemment, l'utilisation d'agents réducteurs conduit à une durabilité médiocre pour le défrisage ou le décrêpage et l'emploi d'hydroxydes, de part leur causticité, limite leur utilisation au domaine du défrisage.

Après d'importantes études, on vient maintenant de découvrir, de façon tout à fait surprenante et inattendue, que l'on pouvait défriser durablement le cheveu en associant l'action d'un dénaturant et d'un moyen de chauffage à une température supérieure à 110°C. On obtient ainsi d'excellents résultats en terme de défrisage, de propriétés cosmétiques des cheveux et d'intégrité de la fibre.

Sans être liée par la théorie, la Demanderesse pense qu'il existe une action conjointe, sur les fibres kératiniques, d'un agent dénaturant et d'un moyen de chauffage, qui permet de les défriser de façon efficace et durable.

La Demanderesse a trouvé qu'il était possible de remédier aux inconvénients de l'art antérieur et de remplir les objectifs précités en mettant en oeuvre un procédé de défrisage des fibres kératiniques comprenant :
- une étape d'application sur les fibres kératiniques d'une composition de défrisage contenant au moins un agent dénaturant de masse moléculaire supérieure à 18,1 g/mol, présent à une concentration molaire comprise entre 1M et 8M,
- une étape d'élévation de la température des fibres kératiniques, à l'aide d'un moyen de chauffage, à une température comprise entre 110 et 250°C, l'agent dénaturant étant une guanidine.

Une concentration molaire comprise entre 1M et 8M correspond à une concentration pondérale comprise entre environ 6 % et environ 80 % par rapport au poids total de la composition.

Ainsi, l'invention a pour objet un procédé de défrisage des cheveux comprenant :
- une étape d'application sur les cheveux d'une composition de défrisage contenant au moins un agent dénaturant de masse moléculaire supérieure à 18,1 g/mol, présent à une concentration molaire comprise entre 1M et 8M,
- puis une étape d'élévation de la température des fibres kératiniques, à l'aide d'un moyen de chauffage, à une température comprise entre 110 et 250°C,
l'agent dénaturant étant une guanidine de formule générale (III) ci-dessous : 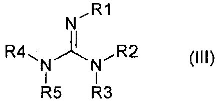 dans laquelle :RI, R2, R3, R4, R5 représentent, indépendamment un atome d'hydrogène ou un radical alkyle ou alcényl inférieur en C1-C4, linéaire ou ramifié, éventuellement substitué par un ou 2 radicaux choisi parmi hydroxyle, amino, diméthylamino, méthoxy, éthoxy, carboxyle, carboxamide, N-méthylcarboxamide ou S03H, lorsque R1, R2 et R3 et R4 représentent un atome d'hydrogène, R5 peut également désigner un radical acétyl; chloroacétyl; carboxamide; méthoxy; éthoxy; 1,2,4-triazolyl; cyclopentyl; méthôxycarbonyle; éthoxycarbonyle; CO-CH=CH-COOH; phényle éventuellement sustitué par un atome de chlore ou un radical hydroxyle; benzyle; thiazolidone; benzimidazole; benzoxazole; benzothiazole; ou C(=NH)-NR6R7 dans lequel R6 et R7 désignent, indépendamment l'un de l'autre, un atome d'hydrogène ou un radical alkyle inférieur en C1-C4, linéaire ou ramifié, éventuellement substitué par un ou 2 radicaux choisi parmi: hydroxyle, amino, diméthylamino, carboxyle ou carboxamide; ou N-méthylcarboxamide; ou encore un radical phényle; lorsque R1=R2=R3=H, R4 et R5 peuvent également former, avec l'atome d'azote qui les porte, un cycle pyrrolidine, pipéridine, pyrazole ou 1,2,4-triazole éventuellement substitués par 1 ou 2 radicaux choisis pami: hydroxyle, amino ou carboxyle; lorsque R1=R2=H, et R4=H ou méthyle, R3 et R5 peuvent également former ensemble un cycle à 5 chainons contenant éventuellement un groupe oxo et leurs sels organiques ou minéraux, caractérisé par le fait que la guanidine de formule (III) constitue le seul actif de défrisage et qu'il est différent de la tétraméthylguanidine.

De préférence, la composition de défrisage comprend entre 2M et 8M dudit agent dénaturant ; ce qui correspond à une concentration pondérale comprise entre environ 12% et environ 80%, par rapport au poids total de la composition, dudit agent dénaturant.

Avantageusement, on élève la température à l'aide du moyen de chauffage à une température comprise entre 120°C et 220°C, plus avantageusement entre 140°C et 220°C.

Avantageusement, la masse molaire du dénaturant est comprise entre 40 et 600 g/mol.

De préférence, ladite composition est appliquée sur des fibres kératiniques humides.

On peut également avantageusement intercaler entre l'étape d'application de la composition et l'étape d'élévation de température, une étape destinée à éliminer l'excédant de la composition, par exemple au moyen d'une serviette.

L'agent dénaturant est une guanidine.

Les documents EP-A-0100901, JP-A-2003212737 et JPH1017441 décrivent des compositions de traitement capillaire comprenant des guanidines.

Par "guanidine", utilisable à titre d'actif de défrisage, on entend tout dérivé comprenant dans sa formule chimique au moins un atome de carbone doublement lié à un atome d'azote et simplement lié à deux autres atomes d'azote. Ces guanidines sont plus sélectionnées parmi les composés de formule générale (III) différent de la tétraméthylguanidine ci-dessous : 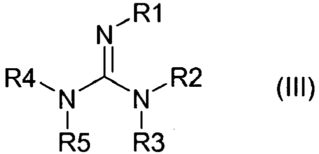 dans laquelle :
R1, R2, R3, R4, R5 représentent, indépendamment, :
   (iii) un atome d'hydrogène ou
   (iv) un radical alkyle ou alcényl inférieur en C1-C4, linéaire ou ramifié, éventuellement substitué par un ou 2 radicaux choisi parmi : hydroxyle, amino, diméthylamino, méthoxy, éthoxy, carboxyle, carboxamide, N-méthylcarboxamide ou SO3H

Lorsque R1, R2 et R3 et R4 représentent un atome d'hydrogène, R5 peut également désigner un radical acétyl ; chloroacétyl ; carboxamide ; méthoxy ; éthoxy ; 1,2,4-triazolyl ; cyclopentyl ; méthoxycarbonyle ; éthoxycarbonyle ; CO-CH=CH-COOH ; phényle éventuellement sustitué par un atome de chlore ou un radical hydroxyle ; benzyle ; thiazolidone ; benzimidazole ; benzoxazole ; benzothiazole ; ou C(=NH)-NR6R7 dans lequel R6 et R7 désignent, indépendamment l'un de l'autre, un atome d'hydrogène ou un radical alkyle inférieur en C1-C4, linéaire ou ramifié, éventuellement substitué par un ou 2 radicaux choisi parmi : hydroxyle, amino, diméthylamino, carboxyle ou carboxamide ; ou N-méthylcarboxamide ; ou encore un radical phényle.

Lorsque R1=R2=R3=H, R4 et R5 peuvent également former, avec l'atome d'azote qui les porte, un cycle pyrrolidine, pipéridine, pyrazole ou 1,2,4-triazole éventuellement substitués par 1 ou 2 radicaux choisis pami : hydroxyle, amino ou carboxyle.

Lorsque R1=R2=H, et R4=H ou méthyle, R3 et R5 peuvent également former ensemble un cycle à 5 chainons contenant éventuellement un groupe oxo

Et leurs sels organiques ou minéraux

Parmi les composés de formule (III), on peut notamment citer les composés préférés suivants :
- la guanidine, chlorhydrate
- la guanidine, acétate
- la guanidine, sulfate
- la guanidine, carbonate
- la guanidine, bicarbonate
- la guanidine, phosphate
- la guanidine, sulfamate
- l'aminoguanidine
- l'aminoguanidine, chlorhydrate
- l'aminoguanidine, sulfate
- l'aminoguanidine, bicarbonate
- la 1,3-diaminoguanidine, chlorhydrate
- la 1-acétylguanidine
- la chloroacétylguanidine, chlorhydrate
- la guanylurée
- la guanylurée, phosphate
- la phénylguanidine, carbonate
- la phénylguanidine, bicarbonate
- la 1-méthylguanidine, chlorhydrate
- la 1,1-diméthylguanidine, chlorhydrate
- la 1-éthylguanidine, chlorhydrate
- la 1,1 -diéthylguanidine, chlorhydrate
- la créatine
- la créatine, monohydrate
- la créatinine, chlorhydrate
- l'agmatine
- l'agmatine, sulfate
- l'acide guanidinoacétique
- l'acide guanidinosuccinique
- l'acide 3-guanidinopropionique
- l'acide 4-guanidinobutyrique
- l'acide 5-guanidinovalérique
- l'acide beta-N-méthylguanidinopropionique
- l'acide N-méthylguanidinopropionique
- la N-(2-hydroxyéthyl)guanidine
- la N-(3-hydroxypropyl)guanidine
- le biguanide, chlorhydrate
- le N-méthyl biguanide, chlorhydrate
- le N-éthyl biguanide, chlorhydrate
- le N-propyl biguanide, chlorhydrate
- le N-butylbiguanide, chlorhydrate
- le 1,1-diméthylbiguanide, chlorhydrate
- le 1-phénylbiguanide
- la 2-tert-butyl-1,1,3,3- tétraméthylguanidine, chlorhydrate
- la L-arginine
- la D-arginine
- la DL-arginine
- l'acide arginique
- la N-amidino-N-(2,3-dihydroxypropyl)glycine
- la N-amidinotaurine
- l'acide 2-imino-1-imidazolidineacétiqué
- la 1-(2,2-diéthoxyéthyl)guanidine
- la 1H-pyrazole-1-carboxamidine, chlorhydrate
- le 5-hydroxy-3-méthyl-1H-pyrazole-1-carboximidamide
- le 3,5-diamino-1H-1,2,4-triazole-1-carboximidamide, chlorhydrate
- la 2-guanidone-4-thiazolidone
- le 2-guanidinobenzimidazole
- le 2-guanidinobenzoxazole
- le 2-guanidinobenzothiazole
- la pyrrolidinoformamidine, chlorhydrate

Parmi les composés de formule (III), on peut notamment citer les composés particulièrement préférés suivants :
- la guanidine, chlorhydrate
- la guanidine, acétate
- la guanidine, sulfate
- la guanidine, carbonate
- la guanidine, bicarbonate
- la guanidine, phosphate
- la guanidine, sulfamate
- l'aminoguanidine, chlorhydrate
- l'aminoguanidine, sulfate
- l'aminoguanidine, bicarbonate
- la 1,3-diaminoguanidine, chlorhydrate
- la guanylurée, phosphate
- la 1-méthylguanidine, chlorhydrate
- la 1,1-diméthylguanidine, chlorhydrate
- la 1-éthylguanidine, chlorhydrate
- la créatine, monohydrate
- la créatinine, chlorhydrate
- l'agmatine
- l'agmatine, sulfate
- l'acide guanidinoacétique
- l'acide guanidinosuccinique
- l'acide 3-guanidinopropionique
- l'acide beta-N-méthylguanidinopropionique
- l'acide N-méthylguanidinopropionique
- la N-(2-hydroxyéthyl)guanidine
- la N-(3-hydroxypropyl)guanidine
- le biguanide, chlorhydrate
- le N-méthyl biguanide, chlorhydrate
- le N-éthyl biguanide, chlorhydrate
- le 1,1-diméthylbiguanide, chlorhydrate
- la 2-tert-butyl-1,1,3,3- tétraméthylguanidine, chlorhydrate
- la L-arginine
- la DL-arginine
- l'acide arginique
- la N-amidino-N-(2,3-dihydroxypropyl)glycine
- la N-amidinotaurine
- l'acide 2-imino-1-imidazolidineacétique
- la 1H-pyrazole-1-carboxamidine, chlorhydrate
- le 3,5-diamino-1H-1,2,4-triazole-1-carboximidamide, chlorhydrate
- la 2-guanidone-4-thiazolidone

Dans les compositions selon l'invention destinées à un processus de défrisage., des cheveux, la guanidine de formule (III) est présente à une concentration molaire comprise entre 1 M et 8M, plus avantageusement, à une concentration comprise entre 2M et 8M.

Le pH des compositions selon l'invention est, de préférence, compris entre 3 et 10, et plus particulièrement compris entre 5 et 9.

Dans les compositions de l'invention, la guanidine de formule (III) constitue le seul actif de défrisage.

Les compositions selon l'invention se présentent soit sous la forme d'une solution aqueuse, soit sous forme d'une crème épaissie de façon à maintenir les cheveux aussi raides que possible. On réalise ces crèmes, sous forme d'émulsions « lourdes ».

Dans le but d'améliorer les propriétés cosmétiques des fibres kératiniques ou encore d'atténuer ou d'éviter leur dégradation, la composition utilisée selon l'invention peut également comprendre un ou plusieurs actifs cosmétiques supplémentaires.

Généralement, le ou lesdits actifs cosmétiques supplémentaires représentent de 0,01 à 30%, de préférence de 0,1 à 10%, en poids du poids total de la composition cosmétique.

Généralement, la composition appliquée sur les fibres kératiniques est appliquée à hauteur de 0,05 à 20 g, de préférence de 0,1 à 10 g de composition par gramme de fibre kératinique sèche.

Après application de la composition, et avant l'élévation de la température des fibres kératiniques au moyen d'un moyen de chauffage, on peut laisser poser ladite composition, généralement pendant 30 secondes à 60 minutes, de préférence 5 à 45 minutes.

Le procédé selon l'invention comprend, après l'étape d'application de la composition, une étape d'élévation de la température des fibres kératiniques, au moyen d'un moyen de chauffage, à une température comprise entre 110°C et 250°C, comme décrit par le document EP-A-1468667.

Avantageusement, on utilise un fer comme moyen de chauffage.

Au sens de la présente invention, on entend par « fer » un dispositif de chauffage des fibres kératiniques mettant en contact lesdites fibres et le dispositif de chauffage.

L'extrémité du fer venant en contact avec les cheveux présente généralement deux surfaces planes. Ces deux surfaces planes peuvent être métalliques. Elles peuvent être lisses ou crantées.

A titre d'exemple de fers utilisables dans le procédé selon l'invention, on peut citer tous types de fer plats et, en particulier, de manière non limitative, ceux décrits dans les brevets US 5 957 140, et US 5 046 516.

L'application du fer peut être effectée par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des fibres kératiniques avant l'étape d'élévation de la température, de manière à éviter d'importants dégagements de vapeurs qui pourraient brûler les mains du coiffeur et le cuir chevelu de la personne. Cette étape de pré-séchage peut se faire par exemple au moyen d'un séchoir, d'un casque, ou bien encore par séchage libre.

L'invention pourra être mieux comprise à l'aide des exemples non limitatifs qui suivent et qui constituent des modes de réalisation préférentiels des compositions selon l'invention.

### EXEMPLE 1:

On réalise une composition de défrisage simplifiée contenant le chlorhydrate de guanidine, à une concentration de 8M dans l'eau, en tant qu'actif de défrisage. Le pH de la composition est de 5,46. On applique cette composition sur des cheveux africains naturellement frisés pendant 15 minutes à une température de 40°C puis on essuie rapidement les cheveux avec une serviette.

On procède ensuite à un lissage mèche à mèche de la chevelure à l'aide d'un fer plat chauffé à 180°C pendant 5 à 10 secondes. Les cheveux sont défrisés efficacement et doux au toucher.

## Revendications

1. Procédé de défrisage des cheveux comprenant :
(i) une étape d'application sur les cheveux d'une composition de défrisage contenant au moins un agent dénaturant de masse moléculaire supérieure à 18,1 g/mol, présent à une concentration molaire comprise entre 1M et 8M,
(ii) une étape d'élévation de la température des cheveux, à l'aide d'un moyen de chauffage, à une température comprise entre 110 et 250°C,
l'agent dénaturant étant une guanidine de formule générale (III) ci-dessous : 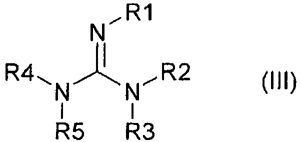 dans laquelle :
R1, R2, R3, R4, R5 représentent, indépendamment un atome d'hydrogène ou un radical alkyle ou alcényl inférieur en C1-C4, linéaire ou ramifié, éventuellement substitué par un ou 2 radicaux choisi parmi : hydroxyle, amino, diméthylamino, méthoxy, éthoxy, carboxyle, carboxamide, N-méthylcarboxamide ou SO3H,
lorsque R1, R2 et R3 et R4 représentent un atome d'hydrogène, R5 peut également désigner un radical acétyl ; chloroacétyl ; carboxamide; méthoxy ; éthoxy; 1,2,4-triazolyl ; cyclopentyl ; méthoxycarbonyle ; éthoxycarbonyle ; CO-CH=CH-COOH ; phényle éventuellement sustitué par un atome de chlore ou un radical hydroxyle ; benzyle ; thiazolidone ; benzimidazole ; benzoxazole ; benzothiazole ; ou C(=NH)-NR6R7 dans lequel R6 et R7 désignent, indépendamment l'un de l'autre, un atome d'hydrogène ou un radical alkyle inférieur en C1-C4, linéaire ou ramifié, éventuellement substitué par un ou 2 radicaux choisi parmi : hydroxyle, amino, diméthylamino, carboxyle ou carboxamide ; ou N-méthylcarboxamide ; ou encore un radical phényle ;
lorsque R1=R2=R3=H, R4 et R5 peuvent également former, avec l'atome d'azote qui les porte, un cycle pyrrolidine, pipéridine, pyrazole ou 1,2,4-triazole éventuellement substitués par 1 ou 2 radicaux choisis pami : hydroxyle, amino ou carboxyle ;
lorsque R1=R2=H, et R4=H ou méthyle, R3 et R5 peuvent également former ensemble un cycle à 5 chainons contenant éventuellement un groupe oxo et leurs sels organiques ou minéraux,
**caractérisé par le fait que** la guanidine de formule (III) constitue le seul actif de défrisage et qu'il est différent de la tétraméthylguanidine.

2. Procédé selon la revendication 1, **caractérisé par le fait que** les composés de formule (III) sont choisis parmi :
- la guanidine, chlorhydrate
- la guanidine, acétate
- la guanidine, sulfate
- la guanidine, carbonate
- la guanidine, bicarbonate
- la guanidine, phosphate
- la guanidine, sulfamate
- l'aminoguanidine
- l'aminoguanidine, chlorhydrate
- l'aminoguanidine, sulfate
- l'aminoguanidine, bicarbonate
- la 1,3-diaminoguanidine, chlorhydrate
- la 1-acétylguanidine
- la chloroacétylguanidine, chlorhydrate
- la guanylurée
- la guanylurée, phosphate
- la phénylguanidine, carbonate
- la phénylguanidine, bicarbonate
- la 1-méthylguanidine, chlorhydrate
- la 1,1-diméthylguanidine, chlorhydrate
- la 1-éthylguanidine, chlorhydrate
- la 1,1-diéthylguanidine, chlorhydrate
- la créatine
- la créatine, monohydrate
- la créatinine, chlorhydrate
- l'agmatine
- l'agmatine, sulfate
- l'acide guanidinoacétique
- l'acide guanidinosuccinique
- l'acide 3-guanidinopropionique
- l'acide 4-guanidinobutyrique
- l'acide 5-guanidinovalérique
- l'acide beta-N-méthylguanidinopropionique
- l'acide N-méthylguanidinopropionique
- la N-(2-hydroxyéthyl)guanidine
- la N-(3-hydroxypropyl)guanidine
- le biguanide, chlorhydrate
- le N-méthyl biguanide, chlorhydrate
- le N-éthyl biguanide, chlorhydrate
- le N-propyl biguanide, chlorhydrate
- le N-butylbiguanide, chlorhydrate
- le 1,1-diméthylbiguanide, chlorhydrate
- le 1-phénylbiguanide
- la 2-tert-butyl-1, 1,3,3- tétraméthylguanidine, chlorhydrate
- la L-arginine
- la D-arginine
- la DL-arginine
- l'acide arginique
- la N-amidino-N-(2,3-dihydroxypropyl)glycine
- la N-amidinotaurine
- l'acide 2-imino-1-imidazolidineacétique
- la 1-(2,2-diéthoxyéthyl)guanidine
- la 1H-pyrazole-1-carboxamidine, chlorhydrate
- le 5-hydroxy-3-méthyl-1H-pyrazole-1-carboximidamide
- le 3,5-diamino-1H-1,2,4-triazole-1-carboximidamide, chlorhydrate
- la 2-guanidone-4-thiazolidone
- le 2-guanidinobenzimidazole
- le 2-guanidinobenzoxazole
- le 2-guanidinobenzothiazole
- la pyrrolidinoformamidine, chlorhydrate

3. Procédé selon l'une quelconque des revendications précédentes, **caractérisé par** le fait la composition de défrisage comprend entre 2M et 8M dudit agent dénaturant.

4. Procédé selon l'une quelconque des revendications précédentes, **caractérisé par le fait qu'**on on élève la température à l'aide du moyen de chauffage à une température comprise entre 120°C et 220°C, plus avantageusement entre 140°C et 220°C.

5. Procédé selon l'une quelconque des revendications précédentes, **caractérisé par le fait que** la masse molaire du dénaturant est comprise entre 40 et 600 g/mol.

6. Procédé selon l'une quelconque des revendications précédentes, **caractérisé par le fait que** la composition est appliquée sur des cheveux humides.

7. Procédé selon l'une quelconque des revendications précédentes, **caractérisé par le fait que** les cheveux sont partiellement préséchés.

8. Procédé selon l'une quelconque des revendications précédentes, **caractérisé par le fait que** l'on applique un fer selon un mouvement continu de la racine à la pointe, en un ou plusieurs passages.

## Patentansprüche

1. Verfahren zum Glätten der Haare, umfassend:
(i) einen Schritt, in dem man auf die Haare eine Glättungszusammensetzung aufbringt, enthaltend mindestens ein Denaturierungsmittel mit einer Molekülmasse von mehr als 18,1 g/mol, das in einer molaren Konzentration zwischen 1 M und 8 M vorliegt,
(ii) einen Schritt, in dem man die Temperatur der Haare mithilfe eines Erhitzungsmittels auf eine Temperatur zwischen 110 und 250°C erhöht,
wobei das Denaturierungsmittel ein Guanidin der nachstehenden allgemeinen Formel (III) ist: 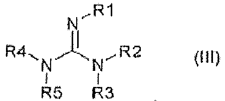 worin:
R1, R2, R3, R4, R5 unabhängig für ein Wasserstoffatom oder einen geraden oder verzweigten C1-C4-Niederalkyl- oder -alkenylrest stehen, der gegebenenfalls mit einem oder zwei Resten substituiert ist, ausgewählt aus: Hydroxyl, Amino, Dimethylamino, Methoxy, Ethoxy, Carboxyl, Carboxamid, N-Methylcarboxamid oder SO3H, wenn R1, R2 und R3 und R4 für Wasserstoff stehen, kann R5 auch einen Acetyl-; Chloracetyl-; Carboxamid-; Methoxy-; Ethoxy-; 1,2,4-Triazolyl-; Cyclopentyl-; Methoxycarbonyl-; Ethoxycarbonylrest; CO-CH=CH-COOH; einen gegebenenfalls mit einem Chloratom oder einem Hydroxylrest substituierten Phenylrest; Benzyl-; Thiazolidon-; Benzimidazol-; Benzoxazol-; Benzothiazolrest; oder C(=NH)-NR6R7 bedeuten, worin R6 und R7 unabhängig voneinander ein Wasserstoffatom oder einen geraden oder verzweigten C1-C4-Niederalkylrest, der gegebenenfalls mit einem oder zwei Resten substituiert ist, ausgewählt aus: Hydroxyl, Amino, Dimethylamino, Carboxyl oder Carboxamid; oder N-Methylcarboxamid; oder auch einem Phenylrest bedeuten;
wenn R1 = R2 = R3 = H, können R4 und R5 auch mit dem Stickstoffatom, das sie trägt, einen Pyrrolidin-, Piperidin-, Pyrazol- oder 1,2,4-Triazolring bilden, die gegebenenfalls mit 1 oder 2 Resten substituiert sind, ausgewählt aus: Hydroxyl, Amino oder Carboxyl;
wenn R1 = R2 = H und R4 = H oder Methyl, können R3 und R5 auch zusammen einen 5-gliedrigen Ring bilden, der gegebenenfalls eine Oxogruppe enthält und deren organische oder anorganische Salze,
**dadurch gekennzeichnet, dass** das Guanidin der Formel (III) den einzigen Glättungswirkstoff darstellt und dass es verschieden von Tetramethylguanidin ist.

2. Verfahren nach Anspruch 1, **dadurch gekennzeichnet, dass** die Verbindungen der Formel (III) aus Folgenden ausgewählt sind:
- Guanidinhydrochlorid
- Guanidinacetat
- Guanidinsulfat
- Guanidincarbonat
- Guanidinhydrogencarbonat
- Guanidinphosphat
- Guanidinsulfamat
- Aminoguanidin
- Aminoguanidinhydrochlorid
- Aminoguanidinsulfat
- Aminoguanidinhydrogencarbonat
- 1,3-Diaminoguanidinhydrochlorid
- 1-Acetylguanidin
- Chloracetylguanidinhydrochlorid
- Guanylharnstoff
- Guanylharnstoffphosphat
- Phenylguanidincarbonat
- Phenylguanidinhydrogencarbonat
- 1-Methylguanidinhydrochlorid
- 1,1-Dimethylguanidinhydrochlorid
- 1-Ethylguanidinhydrochlorid
- 1,1-Diethylguanidinhydrochlorid
- Kreatin
- Kreatinmonohydrat
- Kreatininhydrochlorid
- Agmatin
- Agmatinsulfat
- Guanidinoessigsäure
- Guanidinobernsteinsäure
- 3-Guanidinopropionsäure
- 4-Guanidinobuttersäure
- 5-Guanidinovaleriansäure
- beta-N-Methylguanidinopropionsäure
- N-Methylguanidinopropionsäure
- N-(2-Hydroxyethyl)guanidin
- N-(3-Hydroxypropyl)guanidin
- Biguanidhydrochlorid
- N-Methylbiguanidhydrochlorid
- N-Ethylbiguanidhydrochlorid
- N-Propylbiguanidhydrochlorid
- N-Butylbiguanidhydrochlorid
- 1,1-Dimethylbiguanidhydrochlorid
- 1-Phenylbiguanid
- 2-tert-Butyl-1,1,3,3-tetramethylguanidin-hydrochlorid
- L-Arginin
- D-Arginin
- DL-Arginin
- Argininsäure
- N-Amidino-N-(2,3-dihydroxypropyl)glycin
- N-Amidinotaurin
- 2-Imino-1-imidazolidinessigsäure
- 1-(2,2-Diethoxyethyl)guanidin
- 1H-Pyrazol-1-carboxamidinhydrochlorid
- 5-Hydroxy-3-methyl-1H-pyrazol-1-carboximidamid
- 3,5-Diamino-1H-1,2,4-triazol-1-carboximidamid-hydrochlorid
- 2-Guanidon-4-thiazolidon
- 2-Guanidinobenzimidazol
- 2-Guanidinobenzoxazol
- 2-Guanidinobenzothiazol
- Pyrrolidinoformamidinhydrochlorid.

3. Verfahren nach einem der vorhergehenden Ansprüche, **dadurch gekennzeichnet, dass** die Glättungszusammensetzung zwischen 2 M und 8 M des Denaturierungsmittels umfasst.

4. Verfahren nach einem der vorhergehenden Ansprüche, **dadurch gekennzeichnet, dass** man die Temperatur mithilfe eines Erhitzungsmittels auf eine Temperatur zwischen 120°C und 220°C, besonders vorteilhaft zwischen 140°C und 220°C, erhöht.

5. Verfahren nach einem der vorhergehenden Ansprüche, **dadurch gekennzeichnet, dass** die Molekülmasse des Denaturierungsmittels zwischen 40 und 600 g/mol liegt.

6. Verfahren nach einem der vorhergehenden Ansprüche, **dadurch gekennzeichnet, dass** die Zusammensetzung auf die feuchten Haare aufgetragen wird.

7. Verfahren nach einem der vorhergehenden Ansprüche, **dadurch gekennzeichnet, dass** die Haare zum Teil vorgetrocknet werden.

8. Verfahren nach einem der vorhergehenden Ansprüche, **dadurch gekennzeichnet, dass** man ein Eisen in einer kontinuierlichen Bewegung von der Wurzel bis zur Spitze in einem oder mehreren Durchgängen anwendet.

## Claims

1. Process for relaxing the hair comprising:
(i) a step of applying to the hair a relaxing composition containing at least one denaturing agent with a molecular mass of greater than 18.1 g/mol, present in a molar concentration of between 1M and 8M,
(ii) a step of raising the temperature of the hair, using a heating means, to a temperature of between 110 and 250°C,
the denaturing agent being a guanidine of general formula (III) below: 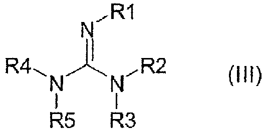 in which:
R1, R2, R3, R4 and R5 represent, independently:
(vii) a hydrogen atom, or
(viii) a linear or branched C1-C4 lower alkyl or alkenyl radical, optionally substituted with one or two radicals chosen from: hydroxyl, amino, dimethylamino, methoxy, ethoxy, carboxyl, carboxamide, N-methylcarboxamide or SO₃H
when R1, R2, R3 and R4 represent a hydrogen atom, R5 may also denote a radical chosen from the following: acetyl; chloroacetyl; carboxamide; methoxy; ethoxy; 1,2,4-triazolyl; cyclopentyl; methoxycarbonyl; ethoxycarbonyl; CO-CH=CH-COOH; phenyl optionally substituted with a chlorine atom or a hydroxyl radical; benzyl; thiazolidone; benzimidazole; benzoxazole; benzothiazole; or C(=NH)-NR6R7 in which R6 and R7 denote, independently of each other, a hydrogen atom or a linear or branched C1-C4 lower alkyl radical, optionally substituted with one or 2 radicals chosen from: hydroxyl, amino, dimethylamino, carboxyl and carboxamide; or N-methylcarboxamide; or alternatively a phenyl radical
when R1=R2=R3=H, R4 and R5 may also form, with the nitrogen atom that bears them, a pyrrolidine, piperidine, pyrazole or 1,2,4-triazole ring, optionally substituted with 1 or 2 radicals chosen from: hydroxyl, amino and carboxyl
when R1=R2=H, and R4=H or methyl, R3 and R5 may also together form a 5-membered ring optionally containing an oxo group
and the organic or mineral salts thereof **characterized in that** the guanidine of formula (III) is the sole relaxing active agent and it is other than tetramethylguanidine.

2. Process according to Claim 1, **characterized in that** the compounds of formula (III) are chosen from:
- guanidine hydrochloride
- guanidine acetate
- guanidine sulfate
- guanidine carbonate
- guanidine bicarbonate
- guanidine phosphate
- guanidine sulfamate
- aminoguanidine
- aminoguanidine hydrochloride
- aminoguanidine sulfate
- aminoguanidine bicarbonate
- 1,3-diaminoguanidine hydrochloride
- 1-acetylguanidine
- chloroacetylguanidine hydrochloride
- guanylurea
- guanylurea phosphate
- phenylguanidine carbonate
- phenylguanidine bicarbonate
- 1-methylguanidine hydrochloride
- 1,1-dimethylguanidine hydrochloride
- 1-ethylguanidine hydrochloride
- 1,1-diethylguanidine hydrochloride
- creatine
- creatine monohydrate
- creatinine hydrochloride
- agmatine
- agmatine sulfate
- guanidinoacetic acid
- guanidinosuccinic acid
- 3-guanidinopropionic acid
- 4-guanidinobutyric acid
- 5-guanidinovaleric acid
- beta-N-methylguanidinopropionic acid
- N-methylguanidinopropionic acid
- N-(2-hydroxyethyl)guanidine
- N-(3-hydroxypropyl)guanidine
- biguanide hydrochloride
- N-methylbiguanide hydrochloride
- N-ethylbiguanide hydrochloride
- N-propylbiguanide hydrochloride
- N-butylbiguanide hydrochloride
- 1,1-dimethylbiguanide hydrochloride
- 1-phenylbiguanide
- 2-tert-butyl-1,1,3,3-tetramethylguanidine hydrochloride
- L-arginine
- D-arginine
- DL-arginine
- arginic acid
- N-amidino-N-(2,3-dihydroxypropyl)glycine
- N-amidinotaurine
- 2-imino-1-imidazolidineacetic acid
- 1-(2,2-diethoxyethyl)guanidine
- 1H-pyrazole-1-carboxamidine hydrochloride
- 5-hydroxy-3-methyl-1H-pyrazole-1-carboximidamide
- 3,5-diamino-1H-1,2,4-triazole-1-carboximidamide hydrochloride
- 2-guanidone-4-thiazolidone
- 2-guanidinobenzimidazole
- 2-guanidinobenzoxazole
- 2-guanidinobenzothiazole
- pyrrolidinoformamidine hydrochloride.

3. Process according to either of the preceding claims, **characterized in that** the relaxing composition comprises between 2M and 8M of the said denaturing agent.

4. Process according to any one of the preceding claims, **characterized in that** the temperature is raised using a means of heating to a temperature of between 120°C and 220°C and more advantageously between 140°C and 220°C.

5. Process according to any one of the preceding claims, **characterized in that** the molar mass of the denaturing agent is between 40 and 600 g/mol.

6. Process according to any one of the preceding claims, **characterized in that** the composition is applied to wet hair.

7. Process according to any one of the preceding claims, **characterized in that** the hair is partially predried.

8. Process according to any one of the preceding claims, **characterized in that** an iron is applied in a continuous movement from the root to the end, in one or more passes.
